Europäisches Patentamt

European Patent Office

Office européen des brevets

(19)

(11) Publication number : **0 547 824 A1**

## (12) EUROPEAN PATENT APPLICATION

(21) Application number : 92311159.5

(22) Date of filing : 08.12.92

(51) Int. Cl.⁵ : **C08L 23/14,** C08L 23/26,
C08L 51/06, C08L 53/00,
A61L 2/08, C08J 7/18,
B29C 71/04, // B29K23/00

(30) Priority : **18.12.91 US 809956**
**28.10.92 US 967837**

(43) Date of publication of application :
**23.06.93 Bulletin 93/25**

(84) Designated Contracting States :
**DE DK FR GB IT SE**

(71) Applicant : **MINNESOTA MINING AND**
**MANUFACTURING COMPANY**
**3M Center, P.O. Box 33427**
**St. Paul, Minnesota 55133-3427 (US)**

(72) Inventor : **Rolando, Richard J., c/o Minnesota**
**Mining and**
**Manufac. Company, 2501 Hudson Road, P.O.**
**Box 33427**
**St. Paul, Minnesota 55133-3427 (US)**
Inventor : **Wilfong, Debra L., c/o Minnesota**
**Mining and**
**Manufac. Company, 2501 Hudson Road, P.O.**
**Box 33427**
**St. Paul, Minnesota 55133-3427 (US)**

(74) Representative : **Baillie, Iain Cameron et al**
**c/o Ladas & Parry, Altheimer Eck 2**
**W-8000 Munich 2 (DE)**

(54) **Mesocopolymers, articles, and methods for preparing same.**

(57)    Mesocopolymers, articles formed from mesocopolymers, and methods for preparing the same are provided. The mesocopolymers and articles are softer and more resistant to degradation by sterilizing dosages of ionizing radiation than comparable crystalline structures, and are expected to be tougher and/or quieter. In addition, the mesocopolymers and articles can include an additional layer grafted thereto by a dose of ionizing radiation.

FIG. 1

EP 0 547 824 A1

## Cross-Reference to Related Applications

This application is a continuation-in-part of pending application Serial No. 07/809,956, filed December 18, 1991.

## Field of the Invention

This invention relates propylene copolymers, articles such as fibers, films, tubes, and nonwoven fabrics formed from propylene copolymers, and to methods for preparing the same.

## Background of the Invention

Polypropylene is often a material of choice for articles of medical use due to its various properties such as non-toxicity and inertness to drugs and liquid media used with drugs, as well as its low cost and the ease with which it can be extruded, molded, and formed into articles. One useful type of polypropylene includes propylene copolymers in which the basic structure of the polymer chain has been changed by the incorporation of a different monomer molecule along with the propylene monomer. In this regard, ethylene and butylene are common monomers that are incorporated along with propylene into such a copolymer chain.

In comparison to polypropylene homopolymers, these propylene copolymers often have improved optical clarity, impact resistance, flexibility, and a decreased melting point. In addition, these copolymers can exhibit important physical properties, such as chemical resistance, water vapor barrier properties, and organoleptic properties (low taste and odor contribution), that are comparable to, if not better than, those exhibited by polypropylene homopolymers. Accordingly, these propylene copolymers are frequently used in blow molding, injection molding, and extrusion applications.

Polypropylene homopolymers, propylene copolymers, and articles manufactured therefrom, generally require sterilization before use in medical or food related applications. A preferred method of sterilization is by gamma radiation using radioactive cobalt, since it can be performed on impermeably wrapped packages, thereby ensuring total and reliable sterility. However, both gamma irradiated polypropylene homopolymers and propylene copolymers are subject to degradation, e.g., embrittlement, discoloration, and thermal sensitivity, during or subsequent to irradiation.

In an attempt to overcome these degradation problems, various stabilizers, such as antioxidants, have been added to polypropylene homopolymers and propylene copolymers in an attempt to prevent discoloration and degradation. For example, U.S. Patent No. 4,110,185 discloses radiation sterilized articles of polypropylene which have incorporated therein a mobilizer which increases the free volume of the polymer and, therefore, lowers the density of the polymer. Suitable mobilizers mentioned include hydrocarbon oils, halogenated hydrocarbon oils, phthalic ester oils, vegetable oils, silicone oils, and polymer greases.

Also, U.S. Patent No. 4,113,595 discloses irradiated crosslinked polyolefin molded products of a blend of a polyolefin, such as polypropylene, a compound having acetylenic linkage, and an aromatic hydrocarbon-substituted organic amine or an aromatic secondary amino compound. Furthermore, U.S. Patents No. 4,274,932 and No. 4,467,065 disclose polypropylene stabilized for irradiation sterilization. The polypropylene has a narrow molecular weight distribution, and has incorporated therein a mobilizer, as used in U.S. Patent No. 4,110,185, described above.

Although the addition of the various stabilizers to polypropylene homopolymers and propylene copolymers serves to diminish degradation by radiation, the use of additives increases costs. In addition, some additives may pose toxicological problems when contacted with pharmaceuticals, while other additives may adversely affect the physical properties of the polypropylene homopolymers and propylene copolymers.

In another attempt to overcome degradation problems, polyethylene has been blended with polypropylene. For example, European Patent Application No. 0,068,555 (Lenzi) discloses radiation-sterilizable polypropylene-based articles, the polypropylene having one to eight weight percent of low density polyethylene added thereto. Specifically, the polyethylene is cross-linked through exposure to radiation, while the polypropylene is allowed to degrade. Thus, it is the cross-liked polyethylene that provides structural integrity for these irradiated articles.

Further attempts have been made to overcome degradation problems associated with crystalline polypropylene. For example, mesomorphous polypropylene, as described in U.S. Patent No. 4,931,230, and articles manufactured from mesomorphous polypropylene, such as described in U.S. Patent No. 4,950,549, provide resistance to sterilizing irradiation. By controlling the method of preparing mesomorphous polypropylene, such as through the quenching of such polypropylene after hot-melt extrusion, the materials or articles formed therefrom substantially maintain their structural integrity after exposure to ionizing radiation at dosages sufficient to degrade crystalline polypropylene. Furthermore, electron beam radiation can also be used to graft additional layers, such as surface adhesion promoting layers, onto films or other structures from such mesomorphous materials. See e.g., U.S. Patent No. 4,950,549.

Unfortunately, packaging films and other articles made from crystalline polypropylene, or even mesomorphous polypropylene, are susceptible to tearing and puncturing which would disrupt the structural integrity of a manufactured component or packaging film after assembly. Thus, the usefulness of a sterilized medical article would be compromised by a puncture or tear in a polypropylene package. In addition, crystalline polypropylene cannot be effectively heat sealed against another material. Furthermore, even though mesomorphous polypropylene provides better heat sealability than crystalline polypropylene, in certain instances it still cannot provide a sufficient heat seal to manufacture a multicomponent medical article, or to provide an effective radiation-sterilized package.

In an effort to overcome these deficiencies, polymer blends of mesomorphous polypropylene and a polymer compatible with such polypropylene, as described in European Patent Application No. 0 405 793 (assigned to the Assignee of the present application) have been developed. These polymer blends exhibit enhanced physical properties, such as heat sealability and tear strength, while maintaining the radiation resistance associated with mesomorphous polypropylene. However, to date, polypropylene in its mesomorphous structure has been limited to mesomorphous polypropylene homopolymer, and a very limited number of compatible polymer blends.

## Summary of the Invention

The present invention provides for mesocopolymers, articles, such as films, fibers, and microfibers manufactured from mesocopolymers, and methods for preparing the same. These mesocopolymers and articles are softer, and more radiation resistant, and are expected to be tougher and quieter than comparable crystalline copolymers. In addition, the surfaces of these mesocopolymers and articles can further include additional graft layers affixed thereto by a dose of ionizing radiation.

In particular, the present invention provides a mesocopolymer comprising a mesophase propylene-based material combined with at least a discernable amount of at least one moiety.

The present invention also can provide a method of preparing a mesocopolymer comprising: (a) heating a copolymer comprising a propylene-based material combined with a discernable amount of at least one moiety to melting; and (b) quenching the heated copolymer immediately after melting to provide a mesocopolymer. In addition, this method can also comprise the further step of irradiating the mesocopolymer with a dosage of ionizing radiation capable of degrading a comparable crystalline copolymer.

In another aspect, the present invention provides an article formed from a mesocopolymer comprising a mesophase propylene-based material combined with at least a discernable amount of at least one moiety. Typically, such articles include films, fibers, microfibers, tubes, and pouches.

In yet another aspect, the present invention provides a method of using an article formed from a mesocopolymer comprising, (a) providing an article formed from a mesocopolymer of a mesophase propylene-based material combined with at least a discernable amount of at least one moiety, and (b) interposing the article between a protected environment and an external environment such that the protected environment remains substantially free from contamination.

For an additional appreciation of the scope of the invention, a more detailed description of the invention follows, with reference to the Drawings.

## Definitions

For the purposes of this invention the definition of "polymer" includes a homopolymer, a copolymer, or an oligomer, as well as any mixtures or blends of one or more homopolymers, and/or one or more copolymers, and/or one or more oligomers.

The term "copolymer" refers to a polymeric material produced by the polymerization of two or more dissimilar monomers, either with or without another functional group, such as maleic anhydride, grafted thereto, as well as to a homopolymer with a functional group grafted thereto. Thus, the term "copolymer" includes, without limitation, random copolymers, block copolymers, sequential copolymers, and graft copolymers.

"Propylene-based material" refers to propylene monomer, or polypropylene polymer.

"Mesophase propylene-based material", refers to a propylene based material in the ordered mesophase form, which is neither amorphous, nor so ordered as to constitute the isotactic I crystalline form (e.g., crystalline polypropylene), such as described by G. Natta et al., "Structure and Properties of Isotactic Polypropylene", Del Nuovo Cimento, Suppl. A1, Vol. XV, Serie X, No. 1, 1960, pp. 40-51, the disclosure of which is herein incorporated by reference. A mesophase propylene-based material is formed by quenching a propylene-based material from the melt state, as defined below, and includes, for example, mesomorphous polypropylene.

The term "moiety" refers to any substance which can be combined with a propylene-based material to form a copolymer, and includes, without limitation, a monomer, a polymer, or a molecule.

"Mesocopolymer"refers to a copolymer of a mesophase propylene-based material and at least a discernable amount of at least one moiety.

"Quenching", refers to the process of immediately and rapidly cooling a copolymer containing a propylene-based material from the melt state such that a mesocopolymer is obtained.

A "graft layer" refers to any additional layer affixed to at least a portion of a mesocopolymer structure of the present invention by grafting a compound or compounds to the surface of the structure through the application of a dosage of ionizing radiation, preferably a dosage of electron beam radiation. For example, a graft layer of acrylic acid (AA) and/or dimethylacrylamide (DMA) can be grafted to at least a portion of the surface of the mesocopolymer structures of the present invention through the exposure of such compound(s) and structures to a dosage of electron beam radiation between about 5 kGY and about 200 kGy. In such an instance, the grafted acrylic acid and/or dimethylacrylamide layer would form a surface adhesion layer that promotes the adhesion of other materials to the modified surface of the structure. Such modification of the surface properties of a mesocopolymer structure is to be contrasted with the bulk properties attributable to, and as a result of, the quenching of a copolymer from the melt state to form a mesocopolymer according to the present invention.

The "structural integrity" of a mesocopolymer can be measured by the percent elongation to break of a structure, such as a film, formed from the mesocopolymer. With respect to radiation resistance of such structures, percent elongation to break is used to measure the extent of degradation or embrittlement of these structures after irradiation. A substantially constant percent elongation at break over several months after irradiation is indicative of substantial maintenance of structural integrity of a mesocopolymer structure over that period after irradiation.

## Brief Description of the Drawing

The invention may be further illustrated by reference to the accompanying Drawings wherein:

FIG. 1 is the wide-angle x-ray diffraction pattern of mesomorphous polypropylene;

FIG. 2 is the wide-angle x-ray diffraction patter of crystalline polyproplyene;

FIG. 3 is the wide-angle x-ray diffraction pattern of the mesocopolymer film of Example 1;

FIG. 4 is the wide-angle x-ray diffraction pattern of the crystalline copolymer film of Comparative Example 8;

FIG. 5 is the wide-angle x-ray diffraction pattern of the mesocopolymer film of Example 2;

FIG. 6 is the wide-angle x-ray diffraction pattern of the crystalline copolymer film of Comparative Example 9;

FIG. 7 is the wide-angle x-ray diffraction pattern of the mesocopolymer film of Example 3;

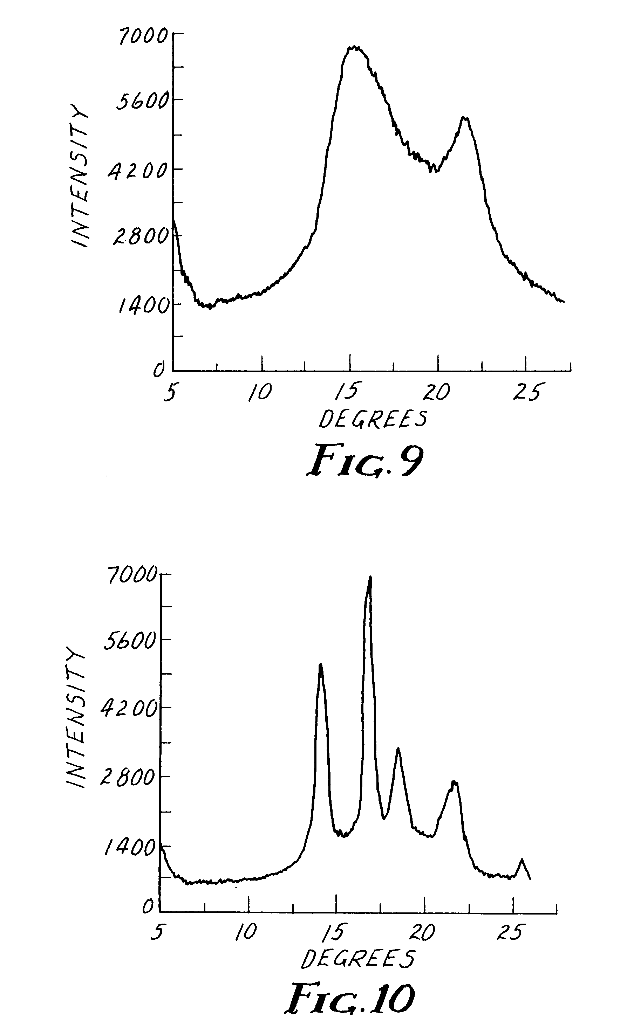

FIG. 8 is the wide-angle x-ray diffraction pattern of the crystalline copolymer film of Comparative Example 10;

FIG. 9 is the wide-angle x-ray diffraction pattern of the mesocopolymer film of Example 4;

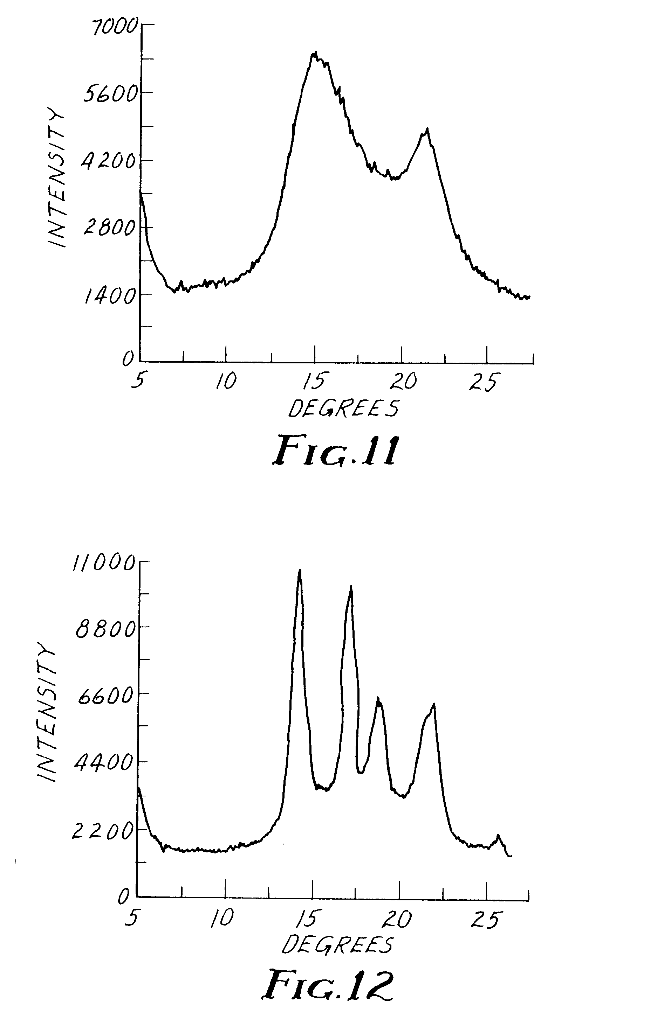

FIG. 10 is the wide-angle x-ray diffraction pattern of the crystalline copolymer film of Comparative Example 11;

FIG.11 is the wide-angle x-ray diffraction pattern of the mesocopolymer film of Example 5;

FIG. 12 is the wide-angle x-ray diffraction pattern of the crystalline copolymer film of Comparative Example 12;

FIG. 13 is the wide-angle x-ray diffraction pattern of the mesocopolymer film of Example 6;

FIG. 14 is the wide-angle x-ray diffraction pattern of the crystalline copolymer film of Comparative Example 13;

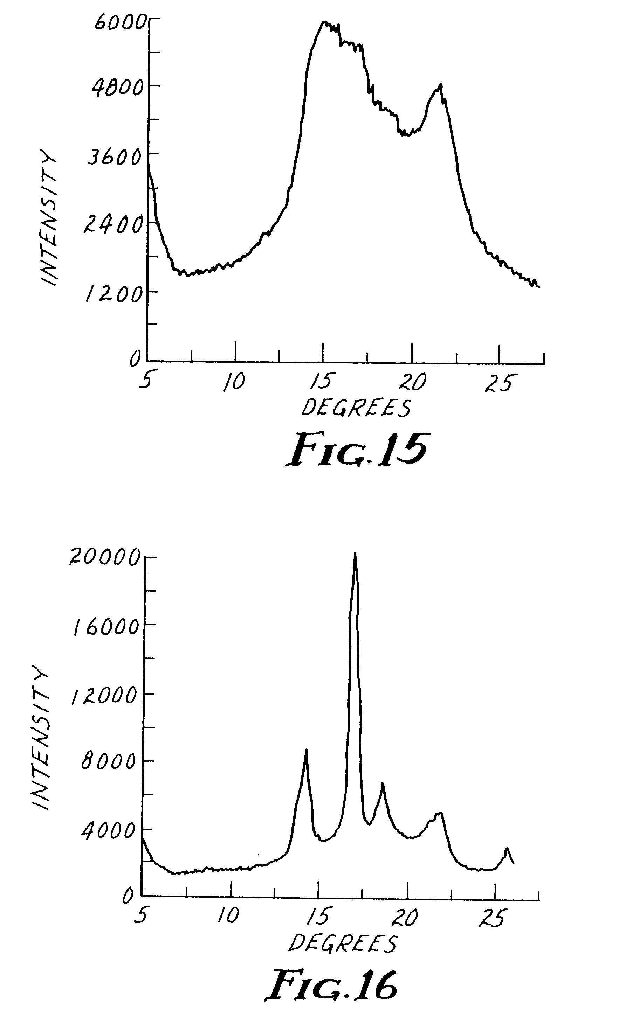

FIG. 15 is the wide-angle x-ray diffraction pattern of the mesocopolymer film of Example 7;

FIG. 16 is the wide-angle x-ray diffraction pattern of the crystalline copolymer film of Comparative Example 14;

FIG. 17 is a graph comparing the radical decay as measured in normalized radical peak height in spins/gram as a function of elapsed time in hows for the mesocopolymer film of Example 1 (line A) and for the crystalline copolymer film of Comparative Example 8 (line B) after exposure to a 50 kGy dosage of electron beam radiation;

FIG. 18 is a graph comparing the radical decay as measured in normalized radical peak height in spins/gram as a function of elapsed time in hours for the mesocopolymer film of Example 3 (line A) and for the crystalline copolymer film of Comparative Example 10 (line B) after exposure to a 50 kGy dosage of electron beam radiation; and

FIG. 19 is the wide-angle x-ray diffraction pattern of the mesocopolymer film of Example 15.

## Detailed Description of Embodiments of the Invention

Previously, it was unknown and unanticipated that copolymers of propylene-based materials in combination with other moieties could or would form mesophase structure upon quenching. However, it has now been surprisingly discovered that copolymers comprised of other moieties (e.g, ethylene) combined with a propylene-based material (e.g., propylene and/or polypropylene) provide mesocopolymers upon quenching. Even more surprisingly, these mesocopolymers can exhibit an overall mesophase structure, even though the other moiety (e.g., ethylene polymer) singly does not exhibit a mesophase structure. In addition, these mesocopolymers are substantially softer, as measured by Young's modulus of elasticity, and more radiation resistant, than a comparable non-quenched crystalline copolymer. Furthermore, it is expected that the mesocopolymers of the present invention will exhibit many, if not all, of the same advantages, including toughness, heat sealability and/or quietness, as mesomorphous polypropylene, blends of mesomorphous polypropylene with compatible and noncompatible polymers, and structures formed therefrom. (See, e.g., U.S. Patent No. 4,931,230, copending U.S. Patent Application Serial No. 07/371,713, and Applicants' copending and co-filed U.S. Patent Applications Serial No. 07/810001, and Attorney Docket Nos. 47008USA3A, 47008USA1B, 47991USA1A, and 47991USA8B, Wilfong et al., all assigned to the Assignee of the present invention, and the disclosures of which are herein incorporated by reference.).

Any moiety, or combination of moieties, can be used in conjunction with mesophase propylene-based materials to form the mesocopolymers according to the present invention. Prior to melt extrusion and quenching, the propylene-based material and other moiety or moieties can be combined in any percent by weight ratio which will result upon quenching in the mesocopolymers of the present invention. Preferably, the optimum weight fraction of mesophase propylene-based material with the other moiety depends upon the intended use and desired properties for the mesocopolymer. Generally, it is desirable to add as much of the other moiety as possible, without compromising the overall mesophase form and its advantageous properties, such as softness and radiation resistance.

However, it is within the scope of this invention to incorporate a discernibly minimal amount of the moiety along with the propylene-based material, quenched to preserve the mesophase form or the mesocopolymers of the present invention. It is expected that such a mesocopolymer would have excellent resistance to sterilizing radiation, and other advantageous physical properties, analogous to that of mesomorphous polypropylene homopolymer.

It is also within the scope of the present invention to incorporate a discernibly minimal amount of propylene-based material along with the moiety to provide a copolymer, quenched to create the mesocopolymer, that would exhibit the advantageous properties of the moiety, such as heat sealability, as well as acceptable radiation resistance.

The weight fraction range of the moiety can be at least ninety nine percent (99%) by weight of the mesocopolymer, more desirably at least seventy five percent (75%) by weight, and more desirable yet, fifty percent (50%) by weight of the mesocopolymer.

Preferably, when it is desirable to balance the best properties of the mesocopolymer, the weight fraction of the moiety should comprise at least forty percent (40%) by weight of the mesocopolymer, more preferably at least twenty percent (20%) by weight, even more preferably at least ten percent (10%) by weight, and most preferably at least five percent (5%) by weight of the mesocopolymer.

The mesocopolymers according to the present invention generally fall within three classes. The first class of copolymer comprises a mesocopolymer wherein the other moiety comprises a monomer, such as ethylene or butylene, that is inserted with propylene monomers in a copolymer chain. Accordingly, class one mesocopolymers of the present invention include, without limitation, random, sequential, and block copolymers. A commercially available example of such a copolymer, which when quenched forms a mesocopolymer according to the present invention, is Petrothane™ resin No. PP7300-KF (quantum Chemical, Inc.). However, it is contemplated that any copolymer that can be formed, for example by a catalyzed or photo-initiated polymerization reaction, melted, and quenched to preserve a mesophase structure, is considered to be within the scope of the present invention.

The second class of mesocopolymers according to the present invention comprise quenched copolymers of the above described class one copolymers, with another moiety grafted to the copolymer chain. For example, the other moiety can comprise a functional group, such as maleic anhydride or acrylic acid, grafted to the copolymer chain, to provide enhanced melt flow rates, as well as other bulk properties. See, e.g., U.S. Patent No. 4,003,874, and British Patent No. 1,393,693, the disclosures of which are herein incorporated by reference. A commercially available example of such a copolymer is Plexar™ resin No. 420 (quantum Chemical, Inc.).

The third, and final, general class of copolymers according to the present invention, which when quenched can provide mesocopolymers, comprise a polypropylene homopolymer with a moiety, such as maleic anhydride or acrylic acid, grafted to the polymer chain. A commercially available example of such a copolymer is Admer™ resin No. QF551A (Mitsui Plastics, Inc.).

In a preferred embodiment, the mesocopolymer comprises a class one copolymer of a propylene monomer combined with a discernable amount of at least one other monomer to form a random, sequential or block copolymer, that is melted and quenched to preserve the mesophase structure. Preferably, the propylene monomer will comprise from about 1% to about 99%, more preferably from about 50% to about 99%, and most preferably from about 90% to about 99% by weight of the mesocopolymer, with the remainder comprising the other monomer, or monomers. The monomers to be combined with propylene to form the mesocopolymers according to the present invention can include any monomer that would polymerize with propylene in the presence of a suitable catalyst, including ethylene, butylene, pentene, methylpentene, and the like. Preferred monomers include ethylene and butylene, with ethylene being particularly preferred.

In a particularly preferred embodiment, the mesocopolymers according to the present invention comprise copolymers of an ethylene monomer with a propylene monomer, quenched to provide the mesocopolymer. Preferably, the ethylene monomer comprises from about 1% to about 25%, more preferably from about 1% to about 20%, and most preferably from about 1% to about 10% by weight of the mesocopolymer, with the remaining monomer comprising propylene.

Many commercially available propylene copolymers which when quenched from the melt state could serve to form mesocopolymers according to the present invention. Nonlimiting examples of such commercially available propylene copolymers include Admer™ resin No. QF551A, a polypropylene graft copolymer (Mitsui Plastics, Inc.; melt index = 5.7 g/10 min); Plexar™ resin No. 420, a polypropylene/polyethylene graft copolymer (Quantum Chemical Corp.; melt index = 2.5 g/10 min.); Shell resin No. 7C05N, a 40% to 60% by weight polypropylene/polyethylene (PP/PE) copolymer combined at 14% by weight with 86% by weight of polypropylene homopolymer (Shell Chemical Corp.; melt index = 15 g/10 min.); PP/PE copolymer resin No. 6571 (Fina Oil and Chemical Co.; melt index = 8 g/10 min.; low ethylene content; melting point (MP) = 148°C); PP/PE copolymer resin No. 7371 (Fina; melt index = 3.5 g/10 min.; medium ethylene content; MP = 143°C); PP/PE copolymer resin No. 8473 (Fina; melt index = 4.6 g/10 min.; high ethylene content; MP = 134°C); and PP/PE copolymer resin No. Z9350 (Fina; melt index = 3.5 g/10 min.; random copolymer; MP = 129°C).

The mesocopolymers according to the present invention may be extruded with other conventional materials to provide for specific additional properties, such as antistatic materials, dyes, plasticizers, ultraviolet absorbers, nucleating agents, and the like. The amount of these materials is typically less than ten weight percent of the mesocopolymer component, preferably less than two percent by weight. However, the mesocopolymers of the present invention do not require any stabilizers, anti-oxidants or the like to enable their mesophase structure, and accordingly, the articles formed therefrom to withstand the effects of ionizing radiation, and still substantially maintain the structural integrity for a useful period of time after irradiation.

The mesocopolymers of the present invention can also be blended with polypropylene homopolymer at from about one percent to about ninety nine percent by weight. A commercially available example of such a mixture is Shell resin No. FC05N, an ethylene-propylene copolymer mixed at a level of 14 percent by weight, to 86 percent by weight of polypropylene homopolymer. As with the mesocopolymer alone, the blend of copolymer and homopolymer must be quenched immediately after extrusion to provide a mesocopolymer component according to the present invention. In addition, the outer surfaces of structures, such as films, formed from the mesocopolymers of the present invention may also desirably have one or more graft layers affixed thereto to enhance other properties, such as surface adhesion, oxygen and/or moisture permeability, coefficient of friction, or other properties desirable to those skilled in the art. For example, not be way of limitation, surface adhesion is desirable in order to provide the application of primers and other coatings that would not otherwise adhere well to the structures of the present invention. In this regard, modification of surface properties of a structure formed from mesocopolymers of the present invention is different and distinct from the bulk properties, such as increased softness and radiation resistance, attributable to the mesocopolymers alone, and due to their melting and quenching according to the methods of the present invention. Thus, the melting and quenching of a propylene graft copolymer, such as Admer™ resin No. QF551A, to provide a mesocopolymer with increased softness and radiation resistance throughout its mass, is to be contrasted from the modification of the surface of an article formed from such a mesocopolymer, by radiation grafting an additional compound or compounds thereon.

Preferably, a graft layer, such as a surface adhesion layer, is grafted to the outer surfaces of mesocopolymer structures by electron beam radiation at dosages of from about 5 kGy (0.5 Mrad) to about 200 kGy (20 Mrad), and preferably at about 50 kGy (5 Mrad), according to the procedures provided in U.S. Patent No. 4,950,549, the disclosure of which is herein incorporated by reference. Nonlimiting examples of compounds that can be grafted to the mesocopolymer structures of the present invention to form a surface adhesion promoting layer include acrylic acid (AA), dimethylacrylamide (DMA), N-vinyl-2-pyrrolidone (NVP), and a copolymer of NVP and trimethylolpropane-triacrylate (NVP/TMPTA). Other potential compounds that can also be used as a graft layer include glycidyl acrylate, hydroxethyl acrylate, hydroxymethyl acrylate, 2-vinyl pyridine, sulfoethyl methacrylate, diisopropylacrylamide, or N,N-diethylamino acrylate. Particularly preferred grafting compounds used to form a surface adhesion layer on a least a portion of these mesocopolymer structures according to the present invention include AA (Aldrich Chemical Co., Milwaukee, WI) and DMA (Chem Service, Inc., Westchester, PA).

The mesocopolymers according to the present invention are substantially softer than comparable non-quenched crystalline copolymers. Specifically, the mesocopolymers exhibit a modulus of elasticity (i.e. Young's modulus measured in Mega Pascals (MPa)) from about one percent to about ninety nine percent less, preferably from about five percent to about fifty percent less than a comparable crystalline structure. In addition the mesocopolymers of the present invention also exhibit increased radiation resistance, and are expect to exhibit, increased toughness (as measured by the fracture strain), and quietness (as measured in Hertz (Hz) of sound emitted), when compared to crystalline propylene copolymers. Specifically, it is expected that the mesocopolymers of the present invention can withstand ionizing radiation dosages of from about 1 kGy (0.1 Mrad) to 200 kGy (20.0 Mrad), and still maintain structural integrity for an extended period of time.

Although mesomorphous polypropylene is known (Natta, G., et al. Structure and Properties of Isotactic Polypropylene, Del Nuovo Cimento Supplemento A1, Volume XV, Serie X, N.1, 1960, pp. 40-51) the present invention for the first time provides mesocopolymers, and articles manufactured therefrom. While not being held to a theory of operation, it is suspected that the radiation stability of the mesocopolymers of the present invention is related to the control of their morphology. Mesomorphous polypropylene has been described as a non-spherulitic structure by P.H. Geil (Polymer Single Crystals, Interscience, N.Y., 1963, p. 270). Conversely, crystalline polypropylene may have "chain-folds", i.e., crystalline/amorphous folds, in the structure which provide areas for radical attack because of their higher energy. In contrast, mesophase structure, such as is present in the mesocopolymers of the present invention, is believed to have ordering as in a Fringed Micelle model, i.e. with no chain-fold defects. It is suspected that this lack of chain fold defects minimizes the number of sites for radical attack and thereby provides the resistance to radiation degradation observed in the mesocopolymers of the present invention.

The mesocopolymers of the present invention can be extruded from the melt state in any shape which can be rapidly cooled throughout after extrusion to obtain a copolymer with a predominantly mesophase form. The shape and/or thickness of the extruded material will be dependent on the efficiency of the quenching systems utilized. Generally, films, tubes, and blown microfiber webs are the preferred extruded materials. The extruded mesocopolymer should not be subjected to temperature treatment that would change the mesophase form of the mesocopolymer to the crystalline phase. After irradiation, the mesocopolymer can be stretched or oriented if properties provided by such treatment are desired.

Various known methods of quenching, as soon as possible, and preferably, immediately after extrusion, can be used to obtain the mesocopolymers of the present invention, including plunging the extruded material into a cold liquid, e.g., ice water bath, spraying the extruded material with a liquid such as water, and/or running the extruded material over a cooled roll or drum.

In a preferred embodiment, the mesocopolymer comprises a film that is preferably quenched by contact with a quench roll, or by plunging the film into a quench bath, such as an ice-water bath. See R.L. Miller, "On the Existence of Near-range Order in Isotactic Polypropylenes", Polymer, 1, 135 (1960), and U.S. Patent No. 4,931,230, both of the disclosures of which are herein incorporated by reference. Where a quench roll is used, the roll temperature is preferably maintained at a temperature below about 38°C, more preferably below about 24°C, and the film is generally in contact with the roll until solidified. The quench roll should be positioned relatively close to the extruder die, the distance being dependent on the roll temperature, the extrusion rate, the film thickness, and the roll speed. Generally, the distance from the die to the roll is about 0.25 cm to about 5 cm. Where a quench bath is used, the bath temperature is preferably maintained at a temperature below about 4°C. The bath should be positioned relatively close to the die, generally about 0.25 cm to about 13 cm from the die to the bath.

In another preferred embodiment, the mesocopolymers comprise melt blown microfibers that are produced by extruding molten copolymer through a die into a high velocity hot air stream to produce fibers having an average fiber diameter of less than about 10 microns. The fibers are collected on a drum in the form of a web. The preparation of microfiber webs is described in Report No. 4364 of the Naval Research Laboratories, published May 25, 1954, entitled "Manufacture of Superfine Organic Fibers," by Wente, Van A. et al., and in Wente, Van A., "Superfine Thermoplastic Fibers" in <u>Industrial Engineering Chemistry</u>, Vol. 48, No. 8, August, 1956, pp. 1342-1346, the disclosures of which are herein incorporated by reference.

To achieve mesocopolymer webs, the blown microfiber web is preferably quenched by spraying with a liquid such as water, or by cooling the collector drum onto which the microfiber web is collected. Optimum quenching can be achieved by spraying the fiber web near the die, then collecting the web on a cooled drum. The water spray is preferably at a temperature of less than about 10°C, less than about 2.5 cm from the die, and the collector drum is preferably about 5 cm to about 10 cm from the die, but can be as much as about 20 cm to about 25 cm, depending on extrusion rates.

In addition, useful articles such as tapes, tubings, containers, films, fibers, microfibers, transdermal drug-delivery patches and various packaging materials can also be formed from the mesocopolymers of the present invention. Thus, articles formed from the mesocopolymers of the present invention are useful to form or cover a protective environment from an external environment, such that the protected environment remains substantially free from contamination, such as for protecting a degradable product contained therein, or a surface covered thereby. For example, such an article can be used to contain a food product or a pharmaceutical product in a protected environment that is substantially free from contamination from the external environment. Similarly, the article can comprise a transdermal drug delivery patch, medical tape, medical tubing, or an ostomy pouch, which protects the body of a mammal, or the body fluids and/or waste products generated by the mammal, from degradation due to contamination from the external environment.

The following non-limiting examples are provided to further illustrate the invention.

## EXAMPLES 1-7, AND COMPARATIVE EXAMPLES 8-14

Fourteen, single-layer extruded films were made using a flat film process. The copolymer resins used were Admer™ number QF551A, a polypropylene graft copolymer (Mitsui Plastics, Inc.; melt index = 5.7 g/10 min); Plexar™ number 420, a polypropylene/polyethylene graft copolymer (Quantum Chemical Corp.; melt index = 2.5 g/10 min.); Shell number 7C05N, a 40% to 60% by weight polypropylene/polyethylene (PP/PE) copolymer combined at 14% by weight with 86% by weight of polypropylene homopolymer (Shell Chemical Corp.; melt index = 15 g/10 min.); PP/PE copolymer number 6571 (Fina Oil and Chemical Co.; melt index = 8 g/10 min.; low ethylene content; melting point (MP) = 148°C); PP/PE copolymer number 7371 (Fina; melt index = 3.5 g/10 min.; medium ethylene content; MP = 143°C); PP/PE copolymer number 8473 (Fina; melt index = 4.6 g/10 min.; high ethylene content; MP = 134°C); and PP/PE copolymer number Z9350 (Fina; melt index = 3.5 g/10 min.; random copolymer; MP = 129°C).

Extrusion melt temperature, screw speed, Wide Angle X-Ray Diffraction (WAXD) structure (i.e., mesomorphous = meso; crystalline = crys), and casting roll temperature for the Example and Comparative Example films are shown in Table 1. The films were generally extruded at a thickness of about 0.05 mm to about 0.075 mm.

| Table 1 | | | | | |
|---|---|---|---|---|---|
| Copolymer resin, extrusion melt temperature, screw speed, WAXD structure, and casting roll temperature for Example films 1-7, and Comparative Example films 8-14. | | | | | |
| Example Number | Copoly Resin Number | Melt Temp. (°C) | Screw Speed (rpm) | WAXD Struct. | Casting Roll Temp. (°C) |
| Ex. 1 | QF551A | 210 | 39 | meso | 18 |
| Ex. 2 | 420 | 249 | 39 | meso | 18 |
| Ex. 3 | 7C05N | 232 | 32 | meso | 16 |
| Ex. 4 | 7371 | 260 | 40 | meso | 18 |
| Ex. 5 | 6571 | 260 | 40 | meso | 18 |
| Ex. 6 | 8473 | 260 | 40 | meso | 18 |
| Ex. 7 | Z9350 | 260 | 40 | meso | 18 |
| Comp. Ex. 8 | QF551A | 210 | 39 | crys | 52 |
| Comp. Ex. 9 | 420 | 249 | 39 | crys | 52 |
| Comp. Ex. 10 | 7C05N | 232 | 32 | crys | 66 |
| Comp. Ex. 11 | 7371 | 260 | 40 | crys | 66 |
| Comp. Ex. 12 | 6571 | 260 | 40 | crys | 66 |
| Comp. Ex. 13 | 8473 | 260 | 40 | crys | 66 |
| Comp. Ex. 14 | Z9350 | 260 | 40 | crys | 66 |

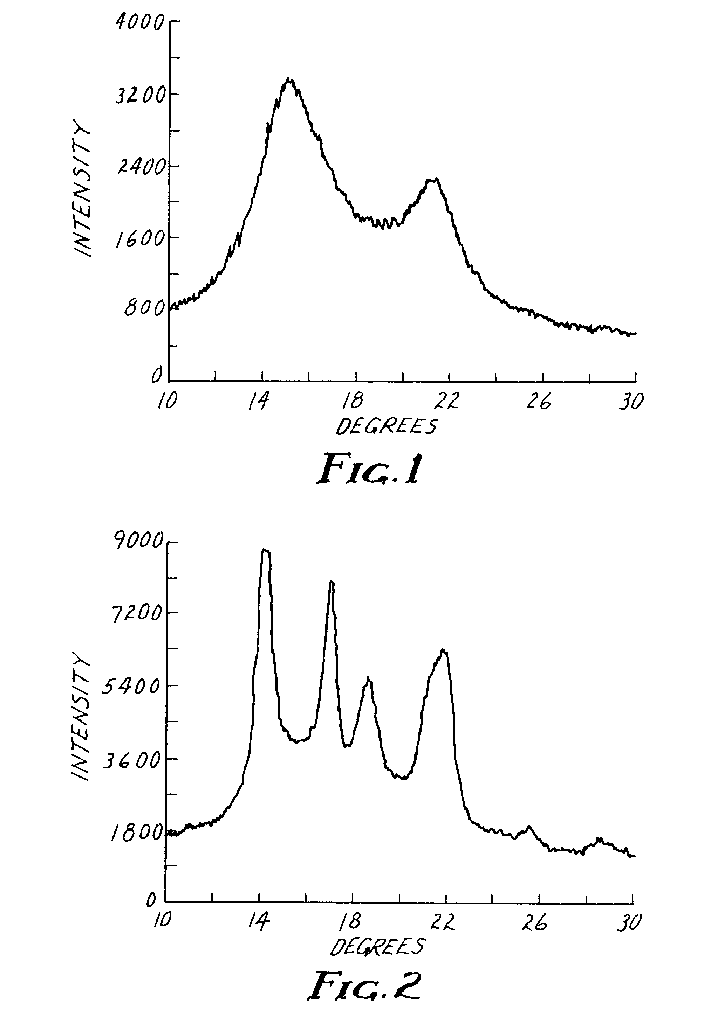

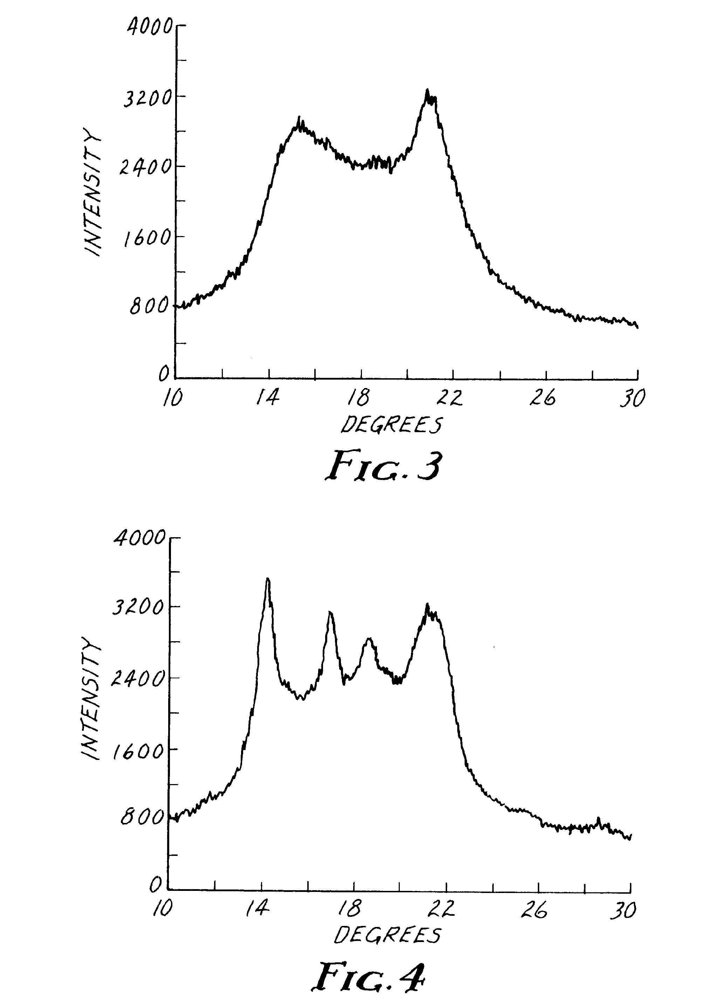

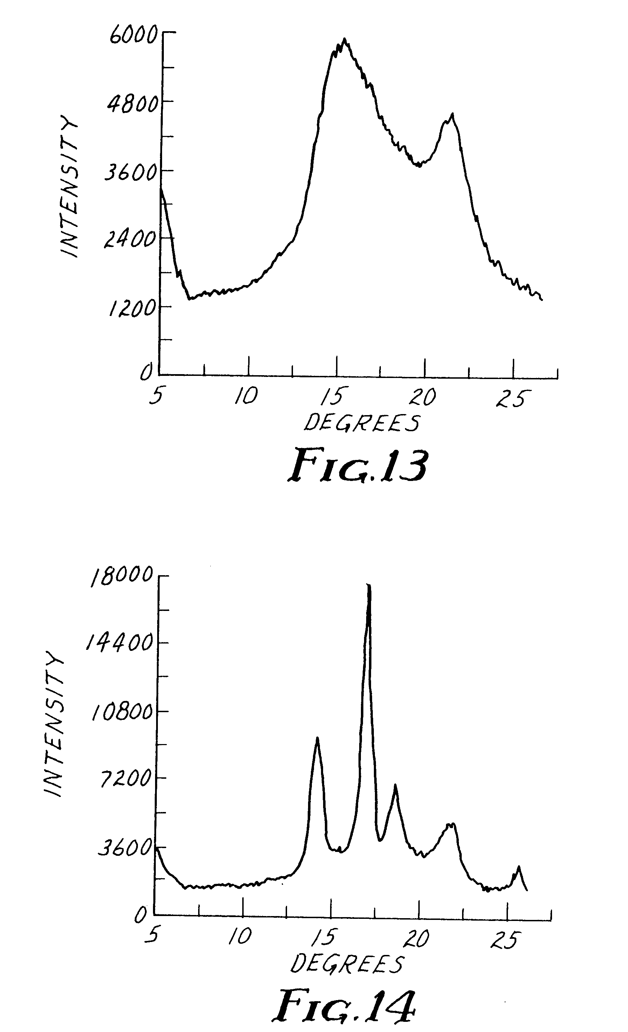

Crystalline or mesophase structures of the single layer films were determined by wide-angle x-ray diffraction (WAXD), and compared to the WAXD of mesomorphous polypropylene homopolymer illustrated in FIG. 1, and of crystalline polypropylene homopolymer illustrated in FIG. 2. Throughout the Figures, the mesophase form of the quenched mesocopolymers of the present invention is readily distinguishable from the crystalline form of the nonquenched copolymer films. For example, the quenched mesocopolymer film of Example 1, illustrated in FIG. 3, has a mesophase form comparable to mesomorphous polypropylene homopolymer (i.e. FIG. 1), while the nonquenched crystalline copolymer film of Comparative Example 8, illustrated in FIG. 4, exhibits an isotactic I crystalline form comparable to that of crystalline polypropylene homopolymer (i.e. FIG. 2). Similarly, the quenched mesocopolymer film of Example 6, illustrated in FIG. 13, clearly displays a mesophase form, while the comparable nonquenched film of Comparative Example 13, illustrated in FIG. 14, shows a crystalline form analogous to that of pure crystalline polypropylene homopolymer (FIG. 2).

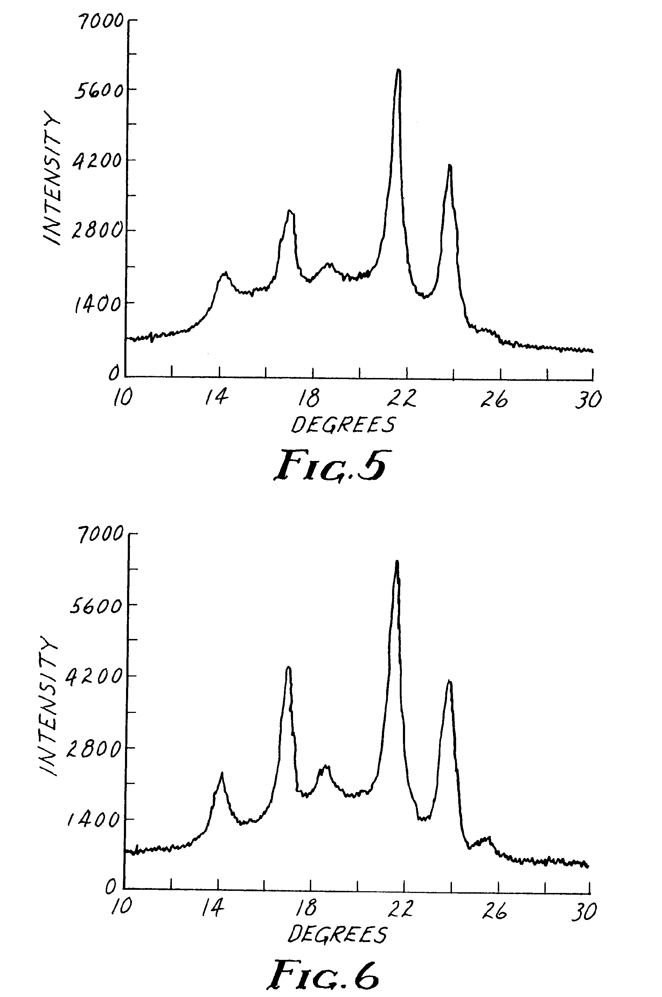

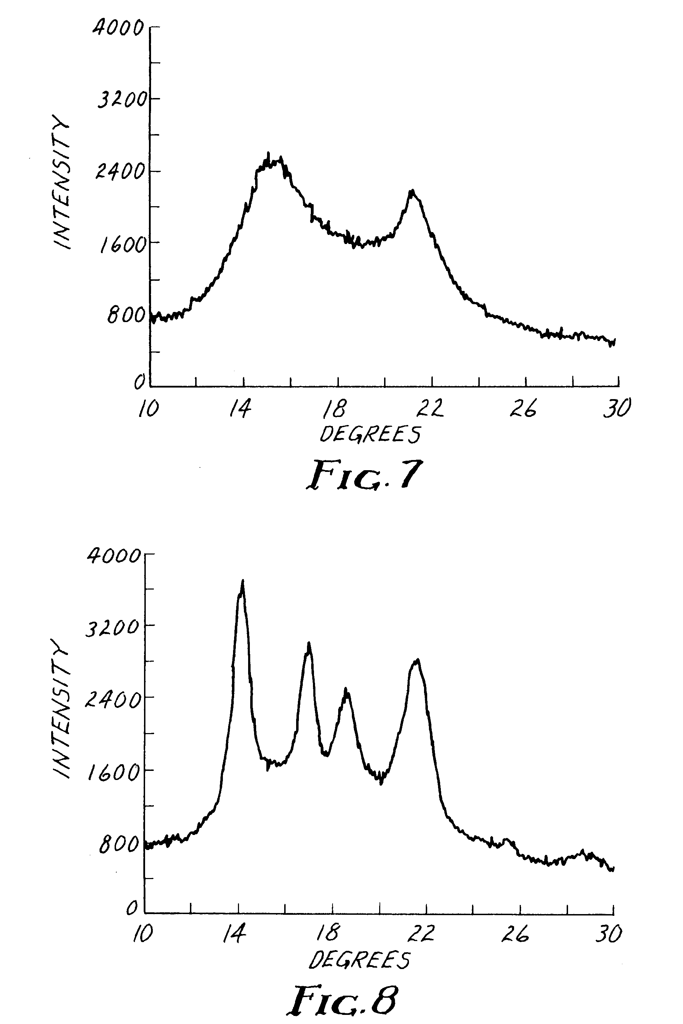

FIGS. 5 and 6 show the WAXD patterns for the quenched mesocopolymer film of Example 2, and the nonquenched crystalline copolymer film of Comparative Example 9. The WAXD pattern in FIG. 5 shows polyethylene as the major crystalline phase along with some isotactic I crystalline structure, as well as a mesophase form of the copolymer. In particular, the inflection at approximately 15° is consistent with the presence of the mesophase form of the mesocopolymer, as illustrated in FIG. 1. The WAXD pattern in FIG. 6 is similar to FIG. 5, except that less, if any, mesophase form is present. Furthermore, the mesophase form of the mesocopolymer of Example 2 was further enhanced by subjecting the molten copolymer to more exacting quenching conditions. See Example 15 and FIG. 19 herein.

Tensile properties were performed on the Example and Comparative Example films using an Instron™ model 1122 machine, using 5 cm x 2.5 cm film samples. Each sample was deformed at a strain rate of one thousand percent (1000%) per minute (sample gauge length of 5 cm and a crosshead speed of 50 cm per minute) using ASTM D882-88 procedures. In each case, at least three samples were measured for each value reported. Table 2 lists modulus values for each of the Example and Comparative Example films. The modulus (Young's modulus, as reported in Mega Pascals (MPa)) for the quenched mesocopolymer containing films of Examples 1-7 is lower than for Comparative Example films 8-14, that contain the comparable crystalline copolymer. Thus, the mesocopolymers of the present invention provide softer structures than comparable crystalline form materials.

| Table 2 | | | |
|---|---|---|---|
| Modulus values (MPa) for Example films 1-7, and Comparative Example films 8-14. | | | |
| Example Number | Copoly Resin Number | Casting Roll Temperature (°C) | Modulus (MPa) |
| Ex. 1 | QF551A | 18 | 179 |
| Ex. 2 | 420 | 18 | 174 |
| Ex. 3 | 7C05N | 16 | 279 |
| Ex. 4 | 7371 | 18 | 238 |
| Ex. 5 | 6571 | 18 | 302 |
| Ex. 6 | 8473 | 18 | 218 |
| Ex. 7 | Z9350 | 18 | 172 |
| Comp. Ex. 8 | QF551A | 52 | 216 |
| Comp. Ex. 9 | 420 | 52 | 193 |
| Comp. Ex. 10 | 7C05N | 66 | 365 |
| Comp. Ex. 11 | 7371 | 66 | 334 |
| Comp. Ex. 12 | 6571 | 66 | 426 |
| Comp. Ex. 13 | 8473 | 66 | 321 |
| Comp. Ex. 14 | Z9350 | 66 | 257 |

The mesocopolymer films of Examples 1 and 3, and crystalline copolymer films of Comparative Examples 8 and 10 were electron beam irradiated at a dosage of 50 kGy (5 Mrads) and then immediately placed in liquid nitrogen. Electron paramagnetic resonance (EPR) analysis was performed by first warming the films to room temperature, cutting them to 1.3 cm x 7.6 cm in size, weighing, and then mounting the film strips in tubes. This technique allows reproducible sample positioning in the EPR cavity. Radical peak heights were recorded for each sample as a function of elapsed time from the initial measurement using a Varian™ model 4502 spectrometer with a 23 cm magnet operating in the "X"-band. Fremy's salt was used as the magnetic field reference. Peak height represents radical concentration as measured in spins/gram. Initial runs were used to estimate radical concentration, and the declining numbers are proportional to the initial number. Since different instrument settings were used for some samples, all numbers were normalized. Spin concentration was calibrated against the National Bureau of Standards No. 261 Ruby Standard.

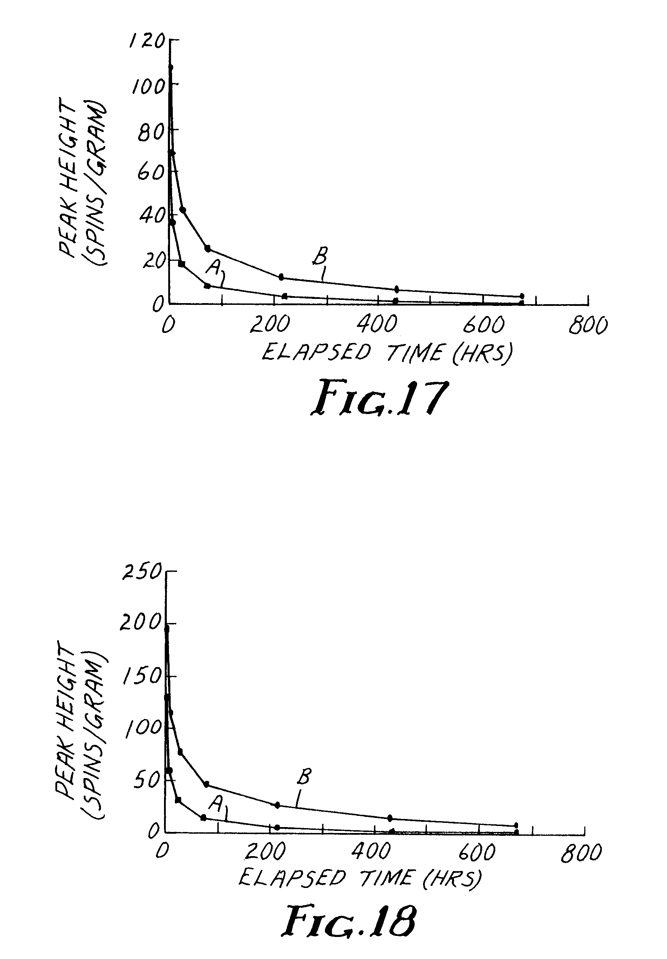

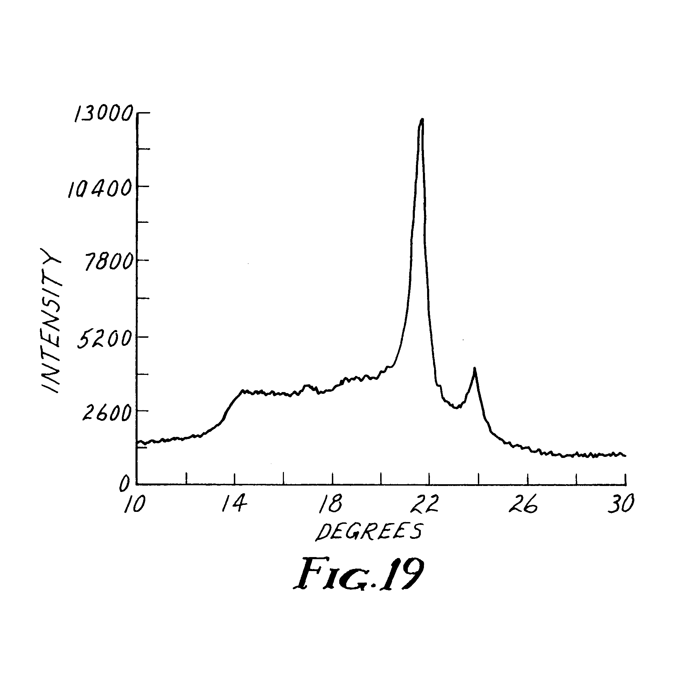

Normalized radical peak height in spins/gram as a function of elapsed time (hours) for the copolymer films is shown in FIGS. 17 and 18. In all cases, radial decay occurs in the quenched films (Examples 1 and 3) at a much faster rate than in the comparison nonquenched films (Comparison Examples 8 and 10). For example, as FIG. 17 illustrates, radical decay occurs at a consistently faster rate for the quenched mesocopolymer film of Example 1 (line A) in comparison to the nonquenched crystalline film of Comparison Example 8 (line B). Likewise, analogous results are shown with the quenched mesocopolymer film of Example 3 (line A) and the nonquenched crystalline copolymer film of Comparison Example 10 (line B) in FIG. 18. Thus, the quenched films comprising the mesocopolymers are expected to maintain their structural integrity and properties to a greater extent than their nonquenched crystalline copolymer counterparts, since the radicals available for degradation are reduced more rapidly in the quenched films than in the nonquenched films.

### Example 15

To enhance the mesophase form of the Plexar™ number 420 copolymer film of Example 2, a 13 cm by 13 cm sample was heated in a hot press to a temperature of 160°C, held for about 3 minutes at that temperature, then instantly plunged into an ice water bath for about 30 seconds. The WAXD of this quenched number 420 film is shown in FIG. 19. This pattern clearly shows a much stronger predominance of the mesophase form than that of FIG. 5. Thus, this pattern shows that the amount of mesophase structure can be controlled by controlling the timing and intensity of the quenching conditions for a given copolymer resin.

## Comparative Examples 16-19 and Examples 20-23

Samples of the mesocopolymer films of Examples 3 and 5 were coated with acrylic acid (AA) monomer (Examples 20 and 22) or dimethylacrylamide (DMA) monomer (Examples 21 and 23). These coated films were then irradiated using an electron beam at a dosage of 50 kGy (5 Mrads) in an inert nitrogen atmosphere, resulting in the grafting of these monomers to the surfaces of the coated films. In addition, two control films each were produced for the Example 3 and Example 5 films. The first control films comprised uncoated and nonradiated samples of the Example 3 and Example 5 films (Comparative Examples 16 and 18). The second control films comprised uncoated films irradiated at a dosage of 50 kGy (Comparative Examples 17 and 19) according to the same procedure as for Examples 20-23. To assess the strength of the grafted layer, 180° peel adhesion measurements were performed according to the following procedure. A 2.5 cm wide, 20.3 cm long strip of pressure-sensitive adhesive tape (Scotch™ brand tape no. 8411; 3M Company) was adhered to a 10.1 cm wide, 15.2 cm long sheet of each of the Example and Comparative Example films, with a free end of the tape extending beyond the end of each film. The sample films were then rolled twice with a 1.35 kg hard rubber roller to ensure contact between the adhesive and the sample films. The samples were then aged at room temperature (22°C) for 24 hows, after which the free end of the tape was removed from the samples at a rate of 15.2 cm/min using a slip/peel testing machine (Instrumentors, Inc., Strongsville, OH). The graft monomers employed, electron beam radiation dosage employed, and the peak peel adhesion force measured in grams/2.5 cm for the sample films of Comparative Examples 16-19, and Examples 20-23 are shown in Table 3.

| Table 3 | | | | |
|---|---|---|---|---|
| Graft monomers used, electron beam radiation dosage employed, and peak peel adhesion measured in grams/2.5 cm for the sample films of Comparative Examples 16-19, and Examples 20-23. | | | | |
| Example Number | Copoly Resin Number | Graft Monomer | E-beam Dose (kGy) | Peak Peel Force (g/2.5 cm) |
| Comp. Ex. 16 | 7C05N | none | 0 | 6.0 |
| Comp. Ex. 17 | 7C05N | none | 50 | 8.2 |
| Ex. 20 | 7C05N | AA | 50 | 12.2 |
| Ex. 21 | 7C05N | DMA | 50 | 12.7 |
| Comp. Ex. 18 | 6571 | none | 0 | 5.7 |
| Comp. Ex. 21 | 6571 | none | 50 | 7.0 |
| Ex. 22 | 6571 | AA | 50 | 12.1 |
| Ex. 23 | 6571 | DMA | 50 | 11.0 |

The data of Table 3 show that mesocopolymer films of the present invention with an additional monomer layer grafted thereto have higher peel strength than the same films lacking in the additional monomer layer, and when not exposed to electron beam radiation in an inert atmosphere. Thus, adhesion promoting layers, as well as other layers, can be added to the mesocopolymer films of the present invention utilizing the above techniques.

## Example 24, and Comparative Example 25

A melt blown mesocopolymer microfiber web according to the present invention (Example 24) could be extruded, as described in Wente, Van A., "Superfine Thermoplastic Fibers", supra, using, for example, the Admer™ number QF551A polypropylene graft copolymer resin. Appropriate extruder conditions for such a resin would be:

Polymer rate (kg/hr)       7
Polymer melt temperature (°C)     329
Air temperature (°C)      343

Air pressure (kPa)                138

The melt blown fibers could then be quenched in a water spray maintained at a temperature of about 4°C, and at a spray rate of about 19 liters/hr, with the spray located about 15 cm below the extrusion die, and directed at the fibers as they exit the die. In addition, or as an alternative method, the web could also be collected on a cooled drum maintained at a temperature of about 4°C, by dipping the roll in ice water, to further quench the web. The webs of Comparative Example 25, would comprise a melt blown microfiber web made by the same process, except that the extruded microfibers would not be quenched. Based on the findings detailed in the specification and Examples herein, it would be expected that the microfibers of Example 24 would be softer, radiation resistant, and have a higher elongation to break than those of Comparative Example 25.

Various modifications and alterations of this invention will be apparent to those skilled in the art without departing from the scope and spirit of this invention and this invention should not be restricted to that set forth herein for illustrative purposes.

## Claims

1. A mesocopolymer comprising a mesophase propylene-based material combined with at least a discernable amount of at least one moiety.

2. A mesocopolymer according to claim 1, wherein the mesophase propylene-based material is selected from the group consisting of propylene monomer, a polypropylene polymer, and combinations thereof.

3. A mesocopolymer according to claim 1, wherein the moiety is selected from the group consisting of a molecule, a monomer, a polymer, and combinations thereof.

4. A mesocopolymer according to claim 3, wherein the monomer is selected from the group consisting of ethylene, butylene, pentene, methylpentene, and combinations thereof.

5. A mesocopolymer according to claim 3, wherein the molecule is selected from the group consisting of an acid molecule, an anhydride molecule, an acid-anhydride molecule, an acetate molecule, an acrylate molecule, and combinations thereof.

6. A mesocopolymer according to claim 5, wherein the acid molecule is selected from the group consisting of acrylic acid, ethylene acrylic acid, and combinations thereof.

7. A mesocopolymer according to claim 5, wherein the anhydride molecule is selected from the group consisting of maleic anhydride, nadic anhydride and combinations thereof.

8. A mesocopolymer according to claim 1, wherein the moiety comprises at least one percent by weight of the mesocopolymer.

9. A mesocopolymer according to claim 1, wherein the moiety comprises at least five percent by weight of the mesocopolymer.

10. A mesocopolymer according to claim 1, wherein the moiety comprises at least ten percent by weight of the mesocopolymer.

11. A mesocopolymer according to claim 1, wherein the moiety comprises at least twenty percent by weight of the mesocopolymer.

12. A mesocopolymer according to claim 1, wherein the moiety comprises at least forty percent by weight of the mesocopolymer.

13. A mesocopolymer according to claim 1, wherein the moiety comprises at least fifty percent by weight of the mesocopolymer.

14. A mesocopolymer according to claim 1, wherein the moiety comprises at least seventy five percent by weight of the mesocopolymer.

15. A mesocopolymer according to claim 1, wherein the moiety comprises at least ninety nine percent by weight of the mesocopolymer.

16. A mesocopolymer according to claim 1, wherein the moiety comprises such an amount that the amount of the mesophase propylene-based material is a discernable amount.

17. A mesocopolymer according to claim 1, wherein the mesophase propylene-based material comprises propylene monomer, and the moiety comprises ethylene monomer.

18. A mesocopolymer according to claim 17, wherein the ethylene monomer comprises from about one percent to about ten percent of the mesocopolymer.

19. A mesocopolymer according to claim 1, wherein the mesocopolymer exhibits a modulus of elasticity substantially less than that of a comparable crystalline copolymer.

20. A mesocopolymer according to claim 1, wherein the mesocopolymer is capable of substantially maintaining its structural integrity after exposure to a dosage of ionizing radiation sufficient to substantially degrade a comparable crystalline copolymer.

21. A mesocopolymer according to claim 1, wherein the mesocopolymer comprises a film, a fiber, a microfiber, a tube, or a pouch.

22. A mesocopolymer according to claim 1, wherein the mesocopolymer further comprises a graft layer affixed to a surface of the mesocopolymer by a dose of ionizing radiation.

23. A mesocopolymer according to claim 1, wherein the graft layer enhances one or more properties of the mesocopolymer, including surface adhesion, coefficient of friction, oxygen permeability, moisture permeability, or combinations thereof.

24. A mesocopolymer according to claim 23, wherein the graft layer comprises a surface adhesion layer formed by affixing to the surface of the mesocopolymer a compound selected from the group consisting of acrylic acid, dimethylacrylamide, N-vinyl-2-pyrrolidone, a copolymer of N-vinyl-2-pyrrolidone and trimethylolpropanetriacrylate, glycidyl acrylate, hydroxyethyl acrylate, hydroxymethyl acrylate, 2-vinyl pyridine, sulfoethyl methacrylate, diisopropylacrylamide, N,N-diethylamino acrylate, and combinations thereof.

25. A method of preparing a mesocopolymer comprising:
a) heating a copolymer comprising a propylene-based material combined with a discernable amount of at least one moiety to melting; and
b) quenching the heated copolymer immediately after melting to provide a mesocopolymer.

26. A method of preparing a mesocopolymer according to claim 25, wherein the quenching occurs at a temperature of less than about 38°C.

27. A method of preparing a mesocopolymer according to claim 25, further comprising, after the quenching step, irradiating the mesocopolymer with a dosage of ionizing radiation that would degrade a comparable crystalline copolymer, wherein the irradiated mesocopolymer remains substantially undegraded.

28. A method of preparing a mesocopolymer according to claim 25, further comprising, after the quenching step, grafting a graft layer to a surface of the mesocopolymer through exposure to a dose of ionizing radiation.

29. A mesocopolymer according to claim 28, wherein the ionizing radiation comprises electron-beam radiation at a dosage of from about 5 kGy to about 200 kGy.

30. A method of preparing a mesocopolymer according to claim 28, wherein the graft layer enhances one or more properties of the mesocopolymer, including surface adhesion, coefficient of friction, oxygen permeability, moisture permeability, or combinations thereof.

31. An article formed from a mesocopolymer comprising mesophase propylene-based material combined with at least a discernable amount of at least one moiety.

13

**32.** An article formed from a mesocopolymer according to claim 31, wherein the article comprises a film, a fiber, a microfiber, a tube, or a pouch.

**33.** An article formed from a mesocopolymer according to claim 31, further comprising a graft layer affixed to a surface of the article by a dose of ionizing radiation.

**34.** An article according to claim 33, wherein the graft layer enhances one or more properties of the articles, including surface adhesion, coefficient of friction, oxygen permeability, moisture permeability, or combinations thereof.

**35.** An article claim 34, wherein the graft layer comprises a surface adhesion layer formed by affixing to the article a compound selected from the group consisting of acrylic acid, dimethylacrylamide, N-vinyl-2-pyrrolidone, a copolymer of N-vinyl-2-pyrrolidone and trimethylolpropanetriacrylate, glycidyl acrylate, hydroxyethyl acrylate, hydroxymethyl acrylate, 2-vinyl pyrridine, sulfoethyl methacrylate, diisopropylacrylamide, N,N-diethylamino acrylate, and combinations thereof.

**36.** A method of using an article formed from a mesocopolymer comprising:
(a) providing an article formed from a mesocopolymer of a mesophase propylene-based material combined with at least a discernable amount of at least one moiety; and
(b) interposing the article between a protected environment and an external environment such that the protected environment remains substantially free from contamination.

**37.** A method of using an article according to claim 36, wherein the article comprises a packaging film containing a degradable product in the protected environment.

**38.** A method of using an article according to claim 37, wherein the degradable product comprises a food product or a pharmaceutical product.

**39.** A method of using an article according to claim 36, wherein the article comprises a transdermal drug delivery patch, a medical tape, medical tubing, or an ostomy pouch.

**40.** A method of using an article according to claim 36, wherein the article further comprises a graft layer affixed to a surface of the article by a dose of ionizing radiation.

FIG.1

FIG.2

FIG. 3

FIG. 4

FIG.5

FIG.6

FIG.7

FIG.8

FIG. 9

FIG. 10

FIG.11

FIG.12

FIG.13

FIG.14

FIG.15

FIG.16

FIG.17

FIG.18

FIG.19

**European Patent Office**

## EUROPEAN SEARCH REPORT

Application Number

EP 92 31 1159

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 431 475 (MITSUI TOATSU CHEMICALS, INCORPORATED) *whole document* | 1-40 | C08L23/14 C08L23/26 C08L51/06 C08L53/00 A61L2/08 C08J7/18 B29C71/04 //B29K23:00 |
| D,A | US-A-4 950 549 (MINNESOTA MINING AND MANUFACTURING COMPANY) *whole document* | 1-40 | |

TECHNICAL FIELDS SEARCHED (Int. Cl.5)

C08L
A61L
C08J
B29C

The present search report has been drawn up for all claims

| Place of search | Date of completion of the search | Examiner |
|---|---|---|
| THE HAGUE | 31 MARCH 1993 | CLEMENTE GARCIA R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)